(19) Europäisches Patentamt
European Patent Office
Office européen des brevets

(11) Publication number: 0 172 380 A2

# (12) EUROPEAN PATENT APPLICATION

(21) Application number: 85108584.5

(22) Date of filing: 10.07.85

(51) Int. Cl.[4]: C 07 C 121/75
C 07 C 120/00

(30) Priority: 20.08.84 US 642297

(43) Date of publication of application: 26.02.86 Bulletin 86/9

(84) Designated Contracting States: BE CH DE FR GB IT LI NL

(71) Applicant: SHELL OIL COMPANY
One Shell Plaza P.O. Box 2463
Houston Texas 77001(US)

(72) Inventor: Petty, Walter L.
2711 Soscol Avenue, 6F
Napa California 94558(US)

(74) Representative: Dreiss, Hosenthien & Fuhlendorf
Gerokstrasse 6
D-7000 Stuttgart 1(DE)

## (54) Preparation of cyanomethyl esters.

(57) Cyanomethyl esters are prepared by reacting a carboxylic acid halide with an alpha-hydroxynitrile in the presence of a minor amount of water, a molar excess of a hydrogen halide acceptor and a catalytic amount of a tertiary-aminopyridine or N-methylimidazole.

EP 0 172 380 A2

K-3524 (EF)
NJG:lh 0172380

# PREPARATION OF CYANOMETHYL ESTERS

## Background of the Invention

Field of the Invention The present invention relates to the preparation of cyanomethyl esters by reacting a carboxylic acid halide with an alpha-hydroxynitrile in the presence of certain amines and a minor amount of water impurity.

Description of the Prior Art It is known to prepare cyanomethyl esters by reacting a carboxylic acid halide with an alpha-hydroxynitrile in the presence of a molar excess of a tertiary amine hydrogen halide acceptor. However, when this reaction is conducted in the presence of minor amounts of water (usually present as impurities in one or more of the ingredients of the reaction), technical difficulties and an unsatisfactorily impure product cyanomethyl ester can arise because free carboxylic acid is formed which then reacts with the carboxylic acid halide to form a relatively unreactive carboxylic acid anhydride byproduct, thereby reducing the yield of desired cyanomethyl ester, which is also contaminated by the byproduct anhydride. The anhydride is not readily removed from the ester by simple conventional procedures, such as extraction or distillation. Thus, it is highly desirable to eliminate this anhydride from the product produced in the presence of water.

## Summary of the Invention

The present invention is directed to a process of preparing a cyanomethyl ester which comprises treating a carboxylic acid halide with an alpha-hydroxynitrile in the presence of a minor amount of water, a molar excess of tertiary amine hydrogen halide acceptor and a catalytic amount of a tertiary aminopyridine or N-methylimidazole. In the presence of these latter catalysts and excess alpha-hydroxynitrile any anhydride that is formed by hydrolysis of the carboxylic acid halide is rapidly converted to the desired ester and an equivalent amount of carboxylic acid, which may be removed by either extraction or distillation. Thus, an ester relatively free of anhydride contamination may be prepared even in the presence of minor amounts of water.

This process of the invention is useful for preparing esters from any optically-active acid halides (which do not contain substituent groups that react with base). For example, the acid halide can be that of an acyclic, alicyclic, aromatic (aryl or aralkyl) or hetero(aromatic) acid.

The reaction is conducted in the absence of a solvent or in the presence of an inert organic solvent, which is suitably selected from non-hydroxylic solvents such as hydrocarbons, chlorinated hydrocarbons, ethers and the like. Suitable solvents are alkanes containing from 5 to 10 carbon atoms such as n-pentane, n-hexane, n-heptane, n-octane, n-nonane, n-decane and their isomers. Petroleum fractions rich in alkanes are also suitable, for example, gasoline with a boiling range at atmospheric pressure of between about 40°C to about 65°C, between about 60°C to about 80°C or between about 80°C to 110°C. Petroleum ether is also suitable. Cyclohexane and methylcyclohexanes are examples of useful cycloalkanes containing from 6 to 8 carbon atoms. Aromatic hydrocarbon solvents can contain from 6 to 10 carbon atoms, for example, benzene, toluene, o-, m- and p-xylene, the trimethylbenzenes, p-ethyltoluene and the like. Suitable chlorinated hydrocarbons contain from 1 to 4 chlorine atoms in combination with an alkane chain containing from 1 to 4 carbon atoms or with a benzene ring, for example, carbon tetrachloride, chloroform, dichloromethane, 1,2-dichloroethane, trichloroethane, perchloroethylene, chlorobenzene and 1,3- or 1,4-dichlorobenzene. Ethers are generally those containing from 4 to 6 carbon atoms such as diethyl ether, methyl tert-butyl ether and diisopropyl ether. Preferably, the solvent is an aromatic hydrocarbon solvent, such as toluene.

The reaction with acid halide is preferably conducted in the presence of a molar excess hydrogen halide acceptor, which is a tertiary amine including a trialkylamine, such as triethylamine, pyridine, N,N-dimethylbenzylamine or 2,6-lutidine, and the like, added slowly, with agitation, and usually after the other reactants are well mixed. Preferably, the halide acceptor is N,N-dimethylbenzylamine.

In the preparation of the cyanomethyl esters by the process of the present invention, it is necessary to have a molar excess of alcohol to acid halide at the end of the reaction in order that any residual anhydride may be converted to ester. Therefore, a molar

ratio of alcohol to acid halide may be from about 10:1 to about 1:1.1 and preferably from about 5:1 to about 1:1.1, the latter ratio depending upon how much acid halide is lost to hydrolysis.

In the preparation of the cyanomethyl ester, the temperature can be varied widely. At normal pressure, for example, the temperature of reaction can suitably be from about 0°C to about 70°C, but is preferably from about 10°C to 40°C more or less.

Separation and recovery of the product cyanomethyl ester are achieved by conventional methods, including crystallization and the like.

The process of the invention is useful for preparing cyanomethyl esters from any carboxylic acid halide which does not contain substituted groups which would react with the base. For example, the acid halides are conventionally known in the art and include an acyclic, alicyclic, aromatic or hetero(aromatic) acid halides and preferably have the formula I

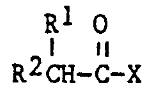

I

wherein X is a halogen atom; $R^1$ and $R^2$ each independently is an alkyl, aralkyl, alkoxy, aryloxy, akylthio, alkylsulfonyl, arylthio, or arylsulfonyl group containing from 1 to 10 carbon atoms or a cycloalkyl group containing 3 to 7 ring carbon atoms, or when taken together with the carbon atom to which they are attached from a cycloalkyl group containing 3 to 7 ring carbon atoms; $R^2$ is also an alkenyl or alkynyl group containing from 2 to 10 carbon atoms; a naphthyl group; a phenyl group; or a heterocyclic group containing 5 or 6 ring atoms, one of which is oxygen, sulfur or nitrogen and the remainder are carbon atoms, or is an amino group disubstituted by one or two acyl groups, or alkyl containing up to 10 carbon atoms. The $R^1$ and $R^2$ groups can be optionally substituted by one or more of halogen of atomic numbers 9 to 35, an alkyl, haloalkyl or cycloalkyl group containing up to 7 carbon atoms, an alkenyl or haloalkenyl group of 2 to 4 carbon atoms, a haloalkoxy or alkoxy group of 1 to 4 carbon atoms, a haloalkylthio or alkylthio group of 1 to 4 carbon atoms or equivalent kinds of substituents. Acid halides of formula I above are preferred in which X is chlorine or bromine.

One class of acid halides are of pyrethroid acids, including those of U.S. patents 4,024,163, 4,062,968, 4,220,591, 3,835,176,

4,243,819, 4,316,913 and 4,199,595. Examples of such acid halides include those having the formula I in which $R^1$ is isopropyl or cyclopropyl; $R^2$ is an alkyl group containing 1 to 6 carbon atoms; an alkenyl group containing 2 to 6 carbon atoms; a naphthyl group, a phenyl group or a (benzyloxycarbonyl)phenylamino group, each optionally ring-substituted by one or more of halogen, alkyl, haloalkyl, alkoxy, haloalkoxy in which the halogens are bromine, chlorine or fluorine and the alkyl groups contain 1 or 4 carbon atoms, or $R^1$ and $R^2$ together with the carbon atom to which they are attached form a cyclopropyl group of the formula

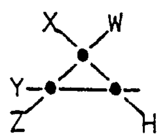

in which W, X, Y and Z each independently is a hydrogen atom, a halogen atom of atomic numbers 9 to 35, or an alkyl group containing 1 to 4 carbon atoms, or Y and Z each independently is an alkyl group containing 1 to 4 carbon atoms, W is a hydrogen atom and X is pentahaloethyl, 2,2-dihalovinyl, isobutenyl, perhalomethylvinyl, 2-phenyl-2-halovinyl, 2-phenyl-1,2,2-trihaloethyl or (alkoxyimino)methyl, or ((cycloalkylalkoxy)imino)methyl of 1 to 10 carbon atoms. For example, the acid halide is isopropyl(4-chlorophenyl)acetyl chloride, isopropyl(4-(difluoromethoxy)phenyl)acetyl chloride, isopropyl((4-trifluoromethyl-3-chlorophenyl)(benzyloxycarbonyl)amino)acetyl chloride, 2,2-dimethyl-3-(2,2-dichlorovinyl)cyclopropanecarbonyl chloride, 2,2-dimethyl-3-(2,2-dibromovinyl)cyclopropanecarbonyl chloride, 2,2-dimethyl-3-(1,2-dibromo-2,2-dichloroethyl)cyclopropanecarbonyl chloride, 1-(4-ethoxyphenyl)-2,2-dichlorocyclopropanecarbonyl chloride, 2,2-dimethyl-3-(2-(trifluoromethyl)-2-chlorovinyl)cyclopropanecarbonyl chloride, 2,2-dimethyl-3-((isobutoxyimino)methyl)cyclopropanecarbonyl chloride, 2,2-dimethyl-3-((neopentoxyimino)methyl)cyclopropanecarbonyl chloride, 2,2-dimethyl-3-(((cyclobutylmethoxy)imino)-methyl)cyclopropanecarbonyl chloride, or chrysanthemyl chloride, and the like.

Preferably, in formula I, $R^1$ is isopropyl and $R^2$ is a phenyl group optionally substituted by halogen, an alkyl or haloalkyl

group of 1 to 4 carbon atoms or an alkoxy or haloalkoxy group containing 1 to 4 carbon atoms, preferably at the para position, especially useful are 4-chlorophenyl, 4-(difluoromethoxyphenyl), 4-methylphenyl, 4-tert-butylphenyl and the like.

Any racemic or optically-active alpha-hydroxynitrile is useful (provided it does not contain substituent groups which would otherwise interfere with the reaction). Preferably, the alpha-hydroxynitrile is a symmetrical or non-symmetrical, racemic or optically-active alpha-hydroxynitrile of formula II

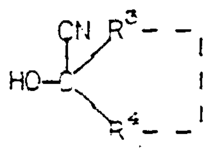

II

wherein $R^3$ is an optionally-substituted hydrocarbyl or heterocyclic group; and $R^4$ is an optionally substituted hydrocarbyl group or a hydrogen atom or $R^3$ and $R^4$ together with the carbon atom to which they are attached form a carbocyclic group as denoted by the dotted line.

The hydrocarbyl groups represented by $R^3$ and $R^4$ in the formula II may be, for example, an alkyl, a cycloalkyl or an aryl group of up to 20 carbon atoms, preferably up to 10 carbon atoms, or $R^3$ in the formula II may be a carbocyclic or an O or S heterocyclic aryl group containing up to 14 carbon atoms. Examples of carbocyclic aryl groups are phenyl, 1-naphthyl, 2-naphthyl and 2-anthryl groups. Heterocyclic aromatic groups are derived from hetero-aromatic compounds which are defined as in Kirk-Othmer, "Encyclopedia of Chemical Technology", Second Edition, Volume 2 (1963), page 702: obtained by replacement of one or more carbon atoms of a carbocyclic aromatic compound by a heteroatom selected from O or S — and also include those heterocyclic compounds having five-membered rings which show aromatic characteristics and are mentioned on page 703 of said volume. Optional substituents include one or more of halogen atoms having an atomic number of from 9 to 35, inclusive, or an alkyl, alkenyl or alkoxy group containing 1 to 6 carbon atoms, each optionally substituted by one or more halogen atoms, optionally substituted phenoxy, phenyl, benzyl or benzoyl and equivalent kinds of substituents.

Illustrative examples of the alpha-hydroxynitriles include alpha-hydroxy-alpha-methylbutyronitrile, alpha-hydroxy-alpha-methylbenzene-acetonitrile, alpha-hydroxyisobutyronitrile and the like.

Preferably, the alpha-hydroxynitrile can be racemic or have the R- or S-configuration, and therefore, include either the racemic, R- or, preferably S-alpha-hydroxynitrile of the formula III

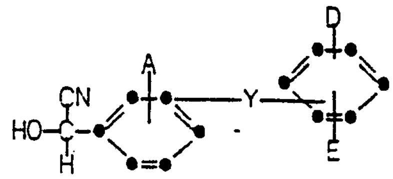

III

wherein Y is O, $CH_2$, or C(O); each A, D and E independently is a hydrogen atom, a halogen atom having an atomic number of from 9 to 35, inclusive, or an alkyl, alkenyl or alkoxy group containing 1 to 6 carbon atoms, each optionally substituted by one or more halogen atoms having an atomic number of from 9 to 35, inclusive. Preferably, each A, D or E independently is a hydrogen atom, a fluorine atom, a chlorine atom, a methyl group, a trifluoromethyl group or a methoxy group. Preferably, one of D and E is a hydrogen atom. An especially preferred subclass of S-alpha-hydroxynitriles are those of the formula above in which D is a hydrogen atom and A and E each independently is a fluorine atom or a hydrogen atom, and, preferably, when either A or E is fluorine, each is located at the 4-position of the ring relative to the benzyl carbon when A or relative to the Y group when E. Especially suitable alcohols are when A is a fluorine atom at the 4-position or a hydrogen atom and E is a hydrogen atom.

Examples of alpha-hydroxynitriles of the above formula include S-alpha-cyano-3-phenoxybenzyl alcohol, S-alpha-cyano-4-fluoro-3-phenoxybenzyl alcohol, S-alpha-cyano-3-(4-fluorophenoxy)benzyl alcohol, and their corresponding enantiomers.

In one embodiment of the invention, an S-alpha-cyano-3-phenoxybenzyl alcohol or mixture enriched therein is treated with an S-alpha-isopropylphenylacetic acid chloride or an optionally-substituted chiral cyclopropanecarboxylic acid chloride to give an optically-active cyanomethyl ester or a mixture enriched therein.

The alpha-isopropylphenylacetic acid halides or a mixture enriched therein are generally known as in U.S. patents 3,996,244 and 4,199,596 and Japanese patents 54/3035 and 54/27,532.

The alpha-hydroxynitriles (cyano alcohols) or a mixture enriched in an optical isomer thereof are known as in U.S. patents 3,835,176 and 4,273,727 or the enriched forms can be prepared from the corresponding racemic alcohols, as for example, when the S-alpha-cyano-3-phenoxybenzyl alcohols or a mixture enriched therein are conveniently prepared from the corresponding aldehyde which is treated with a source of hydrogen cyanide in a substantially water-immiscible, aprotic solvent and in the presence of a cyclo(D-phenylalanyl-D-histidine)dipeptide as catalyst. This process is described in an earlier commonly assigned U.S. patent application, Serial No. 443,763, filed November 22, 1982, now abandoned in favor of continuation-in-part Serial No. 551,548, filed November 14, 1983, and also in Serial No. 535,599, filed September 26, 1983.

The 4-tertiary-aminopyridines and N-methylimidazole are conventional chemicals and are usually used in catalytic amounts of from about 0.01 to about 2.0%w based upon the carboxylic acid halide and, preferably, from about 0.05 to about 1.5%w. It can be useful for the tertiary amine acid halide acceptor to also be the 4-tertiary-aminopyridine or N-methylimidazole. In such cases, the material is present in a molar excess based upon the acid halide. The 4-tertiary-aminopyridines include 4-(dimethylamino)pyridine, 4-(4-methyl-1-piperidinyl)pyridine and the like.

The cyanomethyl esters prepared by one or more embodiments of the process of the invention have the formula IV

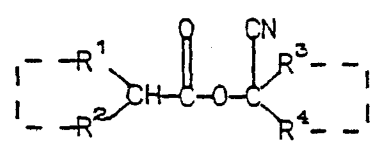

IV

are generally known in the art, including from Francis et al., J. Chem. Soc., 95, pages 1403-1409 (1909) and the like, and in the optical forms, including U.S. patents 4,151,195, 4,239,737, 4,328,167 and 4,133,826, and British patent 2,014,137 and the like. Preferably, the product is an optically-active ester, such as S-alpha-cyano-3-phenoxybenzyl S-alpha-isopropyl-4-chlorophenylacetate, S-alpha-cyano-3-phenoxybenzyl S-alpha-isopropyl-4-(difluoromethoxy)phenylacetate,

alpha-cyano-3-phenoxybenzyl (1R,cis)-3-(1,2-dibromo-2,2-dichloroethyl)-2,2-dimethylcyclopropanecarboxylate, S-alpha-cyano-3-phenoxybenzyl (1R,cis)-2,2-dimethyl-3-(isobutoxyiminomethyl)cyclopropanecarboxylate, and the like or a mixture enriched in such an optically-active ester.

Description of the Preferred Embodiment

Another embodiment of the invention is directed to a process for the preparation of an optically-active cyanomethyl ester or a mixture enriched therein which comprises treating a carboxylic acid halide with an optically-active, optionally substituted alpha-hydroxynitrile (S-alpha-cyanobenzyl alcohol) or mixture enriched herein in the presence of a minor amount of water, a molar excess of a hydrogen halide acceptor and a catalytic amount of a tertiary-aminopyridine or N-methylimidazole to realize the corresponding ester, with retention of optical configuration in the alcohol moiety.

Another embodiment of the present invention is a process for the preparation of an S-alpha-cyano-3-phenoxybenzyl R,S- (or S)-alpha-isopropylphenylacetate in substantially pure form, or of a mixture enriched therein, which comprises treating a racemic or S-alpha-isopropylphenylacetic acid halide with an S-alpha-cyano-3-phenoxybenzyl alcohol or a mixture enriched therein to give the phenylacetate, with retention of optical configuration in the acid and alcohol moieties.

Phenylacetate "alpha" or "A-alpha" products (wherein "alpha" denotes R,S-acid S-alcohol pair and "A-alpha" denotes S-acid S-alcohol single isomer) include those having the formula V

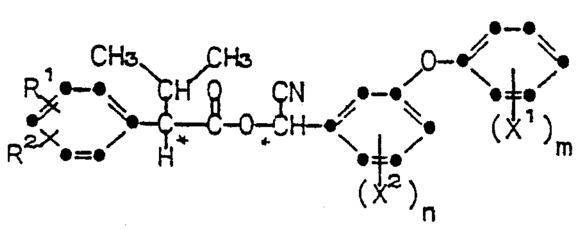

V

wherein $R^1$ is a hydrogen atom, a halogen atom having an atomic number of from 9 to 53, inclusive, or an alkyl group containing from 1 to 4 carbon atoms or an alkoxy group containing from 1 to 2 carbon atoms, each optionally substituted by one or more halogen atoms having an atomic number of from 9 to 53, inclusive, $R^2$ is a hydrogen atom or a methyl group, $X^1$ and $X^2$ each independently is a halogen atom having an atomic number of from 9 to 35, inclusive, or is methyl, and m and

atomic number of from 9 to 53, inclusive, $R^2$ is a hydrogen atom or a methyl group, $X^1$ and $X^2$ each independently is a halogen atom having an atomic number of from 9 to 35, inclusive, or is methyl, and m and n each independently is 0 or 1, and * denotes the asymmetric carbon atom in the acid and alcohol moieties, respectively.

Preferably, $R^1$ is a halogen atom or an optionally halogenated alkyl or alkoxy group as defined above, for example, $R^1$ is a chlorine or fluorine atom, methyl, ethyl, isopropyl, tert-butyl, methoxy, ethoxy, difluoromethoxy or trifluoromethoxy and $R^2$ is a hydrogen atom. $R^1$ is preferably located at the meta- or para-position relative to the benzylic carbon atom in the acid moiety. Preferably, $R^1$ is located at the para-position. Also preferred are those phenylacetates of formula V wherein m is 0 and n is 0 or 1, and $X^2$ is located in the 4-position relative to the benzylic carbon atom in the alcohol moiety. Especially useful are those phenylacetates of formula V wherein n is 0 or when n is 1 then $X^2$ is fluorine at the 4-position. It is further preferred to prepare a phenylacetate product of a material of formula V in which $R^1$ is chlorine or difluoromethoxy, $R^2$ is a hydrogen atom, $X^2$ is fluorine and m is 0 and n is 0 or 1.

Illustrative Embodiments

The following embodiments are provided for the purpose of illustrating the invention and should not be regarded as limiting it in any way. The identity of the products was confirmed by infrared and nuclear magnetic resonance spectral analyses as necessary.

Embodiment 1

A mixture of 2.92 g of R,S-alpha-isopropyl-p-chlorophenylacetic acid chloride and 2.90 g of S-alpha-cyano-3-phenoxybenzyl alcohol containing 0.00715 g of water were combined with 8.9 g of toluene and stirred at 20°C while 1.81 g of N,N-dimethylbenzylamine containing 0.09%w of 4-dimethylaminopyridine was metered in over 1 hour. The reaction mixture was stirred for two additional hours, washed twice with dilute aqueous hydrochloric acid to remove N,N-dimethylbenzylamine hydrochloride and 4-dimethylaminopyridine and stripped of solvent in vacuo to give S-alpha-cyano-3-phenoxybenzyl R,S-alpha-isopropyl-4-chlorophenylacetate containing 1.1%w R,S-alpha-isopropyl-4-chlorophenylacetic anhydride.

The experiment was repeated except in the presence of 0.02715 g of water to give the desired ester containing 0.06%w of anhydride.

Embodiment 2

Following procedures similar to Embodiment 1 above and with the same reactants, 2.92 g of R,S-acid chloride, 3.18 g of S-alpha-cyano alcohol containing 0.00196 g of water were reacted in the presence of the N,N-dimethylbenzylamine as halide acceptor containing the catalytic amount of 4-dimethylaminopyridine to give the desired ester, essentially free of anhydride.

Embodiment 3

A mixture of 2.92 g of R,S-alpha-isopropyl-4-chlorophenylacetic acid chloride and 2.86 g of S-alpha-cyano-3-phenoxybenzyl alcohol containing 0.0218 g of water in 8.4 g of toluene was stirred at 20°C while 1.80 g of N,N-dimethylbenzylamine containing 0.1%w of 4-(4-methyl-1-piperidinyl)pyridine was metered in over 1 hour. The reaction mixture was stirred for one additional hour. The product ester was recovered as described in Embodiment 1 above and contained 0.6%w of anhydride.

Embodiment 4

A mixture of 165.6 g of S-alpha-isopropyl-4-chlorophenylacetic acid, 163.2 g of S-alpha-cyano-3-phenoxybenzyl alcohol and 396.4 g of toluene containing about 0.42 g of water was stirred at 10°C, and a mixture of 104.4 g of N,N-dimethylbenzylamine, 0.11 g of 4-(4-methyl-1-piperidinyl)pyridine, and 250.2 g of toluene containing about 0.93 g of water was metered in during 1 hour. The reaction mixture was stirred for an additional 3 hours. The product S,S ester was recovered as described in Embodiment 1 above and was essentially free of anhydride.

WHAT IS CLAIMED IS:

1. A process for the preparation of a cyanomethyl ester comprises treating a carboxylic acid halide with an alpha-hydroxynitrile in the presence of a minor amount of water, a molar excess of a tertiary amine hydrogen halide acceptor and a catalytic amount of a tertiary-aminopyridine or N-methylimidazole.

2. A process according to claim 1 conducted in the presence of an inert organic solvent.

3. A process according to claim 2 conducted in the presence of an aromatic hydrocarbon and a catalytic amount of a tertiary-aminopyridine.

4. A process according to claim 3 wherein the hydrogen halide acceptor is N,N-dimethylbenzylamine or 4-(4-methylpiperidinyl)pyridine.

5. A process according to claim 3 wherein the acid halide has the formula I

$$R^2\overset{\overset{R^1}{|}}{C}H-\overset{\overset{O}{\|}}{C}-X \qquad I$$

wherein X is a halogen atom or a reactive derivative of the halide, $R^1$ and $R^2$ each independently is an alkyl, aralkyl, alkoxy, aryloxy, alkylthio, alkylsulfonyl, arylthio, or arylsulfonyl group containing from 1 to 10 carbon atoms or a cycloalkyl group containing 3 to 7 ring carbon atoms, or $R^1$ and $R^2$ when taken together with the carbon atom to which they are attached form a cycloalkyl group containing 3 to 7 ring carbon atoms; $R^2$ is also an alkenyl or alkynyl group containing from 2 to 10 carbon atoms; a naphthyl group; a phenyl group; a heterocyclic group containing 5 or 6 ring atoms, one of which is oxygen, sulfur or nitrogen, and the remainder are carbon atoms, or is an amino group disubstituted by acyl, alkyl containing up to 10 carbon atoms. The $R^1$ and $R^2$ groups can be optionally substituted by one or more of halogen of atomic numbers 9 to 35, an alkyl, haloalkyl or cycloalkyl group containing up to 7 carbon atoms, an alkenyl or haloalkenyl group of 2 to 4 carbon atoms, a haloalkoxy or alkoxy group of 1 to 4 carbon atoms, a haloalkylthio or alkylthio group of 1 to 4 carbon atoms.

6. A process according to claim 5 wherein the acid halide of formula I is used in which $R^1$ is isopropyl or cyclopropyl, optionally substituted by one or more chlorine atoms; $R^2$ is an alkyl group containing 1 to 6 carbon atoms; an alkenyl group containing 2 to 6 carbon atoms; a naphthyl group, a phenyl group or a (benzyloxycarbonyl)phenylamino group, each optionally ring-substituted by one or more of halogen, alkyl, haloalkyl, alkoxy, haloalkoxy in which the halogens are bromine, chlorine or fluorine and the alkyl groups contain 1 or 4 carbon atoms; or $R^1$ and $R^2$ together with the carbon atom to which they are attached form a cyclopropyl group of the formula

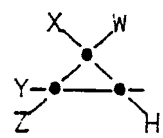

in which W, X Y and Z each independently is a hydrogen atom, a halogen atom of atomic numbers 9 to 35, or an alkyl group containing 1 to 4 carbon atoms, or Y and Z each independently is an alkyl group containing 1 to 4 carbon atoms, W is a hydrogen atom and X is pentahaloethyl, 2,2-dihalovinyl, isobutenyl, perhalomethylvinyl, 2-phenyl-2-halovinyl, 2-phenyl-1,2,2-trihaloethyl or (alkoxyimino)methyl, or (cycloalkylalkoxyimino)methyl of 1 to 10 carbon atoms.

7. A process according to claim 6 wherein in formula I, $R^1$ is isopropyl and $R^2$ is a phenyl group optionally substituted by halogen, an alkyl or haloalkyl group of 1 to 4 carbon atoms or an alkoxy or haloalkoxy group containing 1 to 4 carbon atoms, preferably at the para position, especially useful are 4-chlorophenyl, 4-(difluoromethoxyphenyl), 4-methylphenyl or 4-tert-butylphenyl.

8. A process according to claim 6 wherein the alpha-hydroxynitrile has the formula II

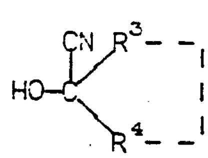

wherein $R^3$ is an optionally substituted hydrocarbyl or heterocyclic group and $R^4$ is an optionally substituted hydrocarbyl group or a hydrogen atom or $R^3$ and $R^4$ together with the carbon atom to which they are attached form a carbocyclic group as denoted by the dotted line.

9. A process according to claim 8 wherein the alpha-hydroxynitrile is alpha-cyano-3-phenoxybenzyl alcohol or alpha-cyano-3-phenoxy-4-fluoro-benzyl alcohol.

10. A process according to claim 9 wherein the acid halide is selected from isopropyl(4-chlorophenyl)acetyl chloride, isopropyl-(4-(difluoromethoxy)phenyl)acetyl chloride, isopropyl(4-(-trifluoromethyl-3-(chlorophenyl)benzyloxycarbonylamino)acetyl chloride, 2,2-dimethyl-3-(2,2-dichlorovinyl)cyclopropanecarbonyl chloride, 2,2-dimethyl-3-(2,2-dibromovinyl)cyclopropanecarbonyl chloride, 2,2-dimethyl-3-(1,2-dibromo-2,2-dichloroethyl)cyclopropanecarbonyl chloride, 1-(4-(ethoxy)phenyl)-2,2-dichlorocyclopropanecarbonyl chloride, 2,2-dimethyl-3-(2-(trifluoromethyl)-2-chlorovinyl)cyclopropanecarbonyl chloride, 2,2-dimethyl-3-((isobutoxyimino)methyl)cyclopropanecarbonyl chloride, 2,2-dimethyl-3-((~~isobut~~neopentoxyimino)methyl)cyclopropanecarbonyl chloride, 2,2-dimethyl-3-(((cyclobutylmethoxyimino)methyl)cyclopropanecarbonyl chloride, or chrysanthemyl chloride.

11. A process according to claim 2 wherein the alpha-hydroxynitrile is alpha-chiral optically-active or a mixture enriched in an alpha-chiral form.

12. A process according to claim 11 wherein a phenylacetate "alpha" or "A-alpha" of an R,S- (or S)-alpha-cyano-3-phenoxybenzyl S-alpha-isopropylphenylacetate in substantially pure form or a mixture enriched therein is prepared by treating an R,S- (or S)-alpha-isopropylphenylacetic acid chloride or a mixture enriched therein with an S-alpha-cyano-3-phenoxybenzyl alcohol or a mixture enriched therein in the presence of a hydrocarbon solvent, a molar excess of a tertiary amine hydrogen halide acceptor and a tertiary-aminopyridine.

13. A method according to claim 12 wherein the S-alpha-cyano-3-phenoxybenzyl R,S- (or S)-alpha-isopropylphenylacetate or a mixture enriched therein has a formula V

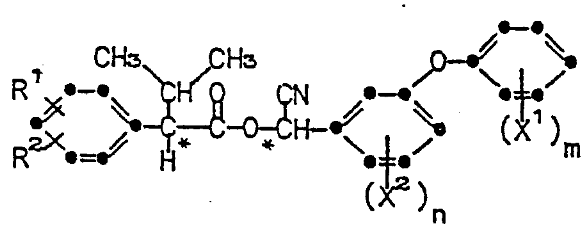

V

wherein $R^1$ is a hydrogen atom, a halogen atom having an atomic number of from 9 to 53, inclusive, or an alkyl group containing from 1 to 4 carbon atoms or an alkoxy group containing from 1 to 2 carbon atoms,

each optionally substituted by one or more halogen atoms having an atomic number of from 9 to 53, inclusive; $R^2$ is a hydrogen atom or a methyl group; $X^1$ and $X^2$ each independently is a halogen atom having an atomic number of from 9 to 35, inclusive, or is methyl; m and n each independently is 0 or 1 and each * denotes the asymmetric carbon atom in the acid and alcohol moieties, respectively.

14. A method according to claim 13 wherein $R^1$ is a hydrogen atom, $R^2$ is a chlorine atom, m is 0, n is 0 or 1 and $X^2$ is a fluorine atom at the 4-position relative to the benzylic carbon atom.

15. A method according to claim 12 wherein the tertiary aminopyridine is 4-dimethylaminopyridine or 4-(4-methyl-1-piperidinyl)pyridine.